# EUROPEAN PATENT APPLICATION

(11) **EP 4 806 455 A1**
(43) Date of publication of application: **16.09.2026**
(21) Application number: 25163267.5
(22) Date of filing: 12.03.2025
(51) Int. Cl.: A61K 38/18, A61P 27/06

(54) **NGF FOR THE USE IN SLOWING THE PROGRESSION OF GLAUCOMA AND/OR DELAYING THE ONSET OF GLAUCOMA**

(71) Applicant: Dompé farmaceutici SpA, 20122 Milano (IT)
(72) Inventor: CYPRIANO DUTRA, Rafael, 67100 L'Aquila (IT); ARAMINI, Andrea, 67100 L'Aquila (IT); CATTANI, Franca, 67100 L'Aquila (IT); VECCHIOTTI, Davide, 67100 L'Aquila (IT); ZAZZERONI, Francesca, 67100 L'Aquila (IT); CIMINI, Annamaria, 67019 L'Aquila (IT); TESSITORE, Alessandra, 67100 L'Aquila (IT); ALESSE, Edoardo, 67100 L'Aquila (IT); ALLEGRETTI, Marcello, 67100 L'Aquila (IT)
(74) Representative: Dompé farmaceutici Spa

(57) **Abstract**

The present invention relates to nerve growth factor (NGF) for use in slowing the progression of vision function impairment caused by glaucoma, and/or delaying the onset of vision function impairment or vision function loss caused by glaucoma in a subject, wherein said NGF is administered intravitreally to said subject.

## Description

### FIELD OF THE INVENTION

The present invention relates to NGF for use in slowing the progression of vision function impairment caused by glaucoma, and/or delaying the onset of vision function impairment or vision function loss caused by glaucoma in a subject.

### STATE OF THE ART

Glaucoma, one of the leading causes of blindness throughout the world, encompasses a group of ocular degenerative disorders characterized by progressive retinal ganglion cells degeneration and optic neuropathy.

A number of mechanisms have been invoked to explain RGC degeneration in glaucoma, including chronic intermittent ischemia, oxidative stress, inflammation, excitotoxicity, defective axon transport, trophic factor withdrawal, and loss of electrical activity (Chang EE et al, Ophthalmology 2012 May, 119(5): 979-86).

The progressive death of retinal ganglion cells (RGCs), the output neurons of the retina, manifests clinically as a thinning of the retinal layer, including nerve fiber layer (RNFL; RGC axons), the ganglion cell layer (GCL; RGC somas), and inner plexiform layer (IPL; RGC dendrites), and cupping/ excavation of the optic nerve head (ONH; where RGC axons bundle and exit the eye as the optic nerve).

Besides RGC apoptosis, oxidative stress and inflammation also affect glaucomatous retina cells, which contribute to the worsening of the correct and complete function thereof. In particular, the reactive gliosis markers Iba-1 (ionized calcium-binding adapter molecule 1), and GFAP (Glial fibrillary acidic protein) are reported to be significantly increased in the retina and optic nerve head of the glaucomatous eyes, thus indicating that significant neuroinflammation is associated with the glaucomatous disease (Rutigliani, C. et al, Acta Neuropathol Commun. 2022 Aug 19;10(1):118).

In most cases, glaucoma is associated with an inadequate drainage of aqueous humor through outflow pathways. Poor drainage results in the accumulation of aqueous humor and may cause IOP elevation, which contributes to irreversible, progressive damage to retinal ganglion cells (RGC) and resultant retinopathy and optic neuropathy.

Based on the anterior chamber anatomy and mechanism of aqueous humor outflow impairment, glaucoma is mainly classified in two main categories:
▪ open-angle glaucoma, with optic nerve damage associated with an open irido-corneal drainage angle. This is usually associated with an increased resistance to aqueous outflow through the trabecular meshwork. Intraocular pressure (IOP) is usually elevated but in some cases may be within the average range (normal-tension glaucoma). This type of glaucoma is typically asymptomatic in early stages and progresses slowly.
▪ angle-closure glaucoma, with optic nerve damage associated with a physical obstruction of the irido-corneal drainage angle. This type of glaucoma can be acute, where a sudden and severe obstruction and increase in IOP causes pain and vision loss, or chronic, with a gradual angle narrowing and increase in IOP leading to a progressive visual function degeneration.

These two types of glaucoma can then be further classified based on their underlying cause into primary, when no cause of outflow blockage is identified or secondary, when the outflow obstruction results from an identifiable cause, such as trauma, inflammation, neovascularization, or medication use.

In a patient with a diagnosis of glaucoma, progression and severity of the pathology may be evaluated on the basis on the abnormalities in the visual field, measured via the Humphrey 24-2 visual field (VF) test.

According to this classification system, the following four stages of glaucoma can be identified:
- Early stage (also known as mild glaucoma), with a mean deviation in the Humphrey 24-2 visual field (VF) test better than -6dB. This stage is characterized by minimal or not detectable vision loss.
- Intermediate stage (also known as moderate glaucoma) with a mean deviation in the Humphrey 24-2 visual field (VF) test between -6.01 and -12 dB. This stage is characterized by mild to moderate visual impairment, with preservation of central vision.
- Advanced stage (also known as severe glaucoma), with a mean deviation in the Humphrey 24-2 visual field (VF) test between -12.01 and -20 dB. This stage is characterized by noticeable visual impairment, including difficulty with contrast sensitivity and night vision.
- Late stage (also known as end stage glaucoma) with a mean deviation in the Humphrey 24-2 visual field (VF) test worse than -20.01 dB. This stage is characterized by severe vision loss, tunnel vision and legal blindness.

Currently, the only existing treatment for glaucoma consists in reducing the intraocular pressure (IOP). This is achieved by topical pharmacological treatments effective in lowering intraocular pressure via different mechanisms of action, or by the more invasive laser or incisional surgeries to improve aqueous outflow or establish a new outflow pathway for the aqueous humor (Weiner et al, JAMA 2014 14; 311(18): 1901-1911, Bedrood S. et al., Clin Ophthalmol. 2023 14;17: 3899-3913).

The above approach aims to block or slow progression of the disease and prevent further optic nerve and visual field damage. However, in many patients glaucoma progression still continues even after a significant IOP reduction has been achieved.

Therefore, there is a great unmet need for the development of new therapeutic approaches effective in attaining vision preservation or restoration in glaucoma patients, in particular protecting of RGC from degeneration.

The development of new therapies for glaucoma is particularly challenging also due to the fact that some administration routes are generally not indicated for treating glaucoma, in order to avoid the risk of worsening visual loss, and/or accelerating the degenerative process of this disease; for example, caution is advised with the use of the intravitreal administration route as this may cause acute and chronic postinjection IOP increase, which may promote glaucomatous damage in susceptible optic nerves (Levin AM., et al., J Glaucoma. 2021 Dec 1;30(12):1019-1026).

In the light of the above, it is strongly felt the need to develop new effective and safe therapeutic approaches to slow the progression of vision function impairment caused by glaucoma, and/or delay the onset of vision function impairment or vision function loss caused by glaucoma in a subject.

### SUMMARY OF THE INVENTION

As it will described in the experimental section, the present inventors have now demonstrated that the intravitreal administration of Nerve Growth Factor (NGF) unexpectedly protects the eye cells, in particular the retinal cells, from several types of damage caused by glaucoma.

Said damage may be caused by inflammation and/or oxidative stress, due to said glaucoma disease, of the retinal cells.

In particular, by a histochemical point of view, said NGF treatment causes an increase of the thickness of the total retina, as well as the thickness of each of the retinal layers, thus counteracting the trend of glaucomatous retina to become thinner with the progression of the disease.

Moreover, the intravitreal administration of NGF also surprisingly determines an increase of the RGC count and vitality in glaucomatous retinas.

Said NGF treatment also unexpectedly shows a significant neuroprotective effect on the glaucomatous retinal cells, as it causes an increase of the expression of anti-oxidant genes, and the down-regulation of pro-oxidant genes in said cells.

Furthermore, said NGF treatment also demonstrates a significant cytoprotective anti-inflammatory effect on the glaucomatous retinal cells, by causing an increase of the expression of anti-inflammatory genes, and the down-regulation of pro-inflammatory genes in said cells.

Surprisingly, the intravitreal administration of NGF does not cause an increase in the intraocular pressure (IOP), even 4 weeks after the administration, in the early, intermediate and advanced stages of glaucoma, as defined in the present invention .

Accordingly, a first object of the invention is nerve growth factor (NGF) for use in slowing the progression of vision function impairment caused by glaucoma, and/or delaying the onset of vision function impairment or vision function loss caused by glaucoma in a subject, said NGF being administered intravitreally to the subject.

A further object of the invention is a pharmaceutical composition comprising nerve growth factor (NGF), and at least one pharmaceutically acceptable excipient, for use in slowing the progression of vision function impairment caused by glaucoma, and/or delaying the onset of vision function impairment or vision function loss caused by glaucoma in a subject, said pharmaceutical composition being administered intravitreally to the subject.

Said subject is a subject suffering from glaucoma, preferably in the early, intermediate and advanced stages of glaucoma, as defined in the present invention, or a subject susceptible to developing glaucoma.

### BRIEF DESCRIPTION OF THE FIGURES

**Figure 1** shows the thickness (µm) of total retina (A), the thickness (µm) of the NFL + GCL, IPL, INL, OPL, ONL, PL retinal layers (B), the RGCs count (normalized to area) (C), the percentage of IBA1 positive cells (D), percentage of GFAP positive cells (E), and the TUNEL test results, of vehicle-treated ("Vehicle"), and rhNGF-treated ("rhNGF") glaucoma mice, according to Example 1 (*p< 0.05; , **p< 0.01 , ***p< 0.001).
**Figure 2** shows the (normalized) expression level of Pou4f1/Brn3a (A), Sncg (B), Rbpms (C), UCHL1 (D), Tubb3 (E), Rbpms2 (F), Elavl3 (G) and Nefl (H) genes in vehicle-treated ("Vehicle"), and rhNGF-treated ("rhNGF") glaucoma mice, according to Example 1 (*p< 0.05, **p< 0.01, ***p< 0.001).
**Figure 3** shows the expression level (normalized) of NOS1 (A), NOS2 (B), and NOS3 (C) genes in vehicle-treated ("Vehicle"), and rhNGF-treated ("rhNGF") glaucoma mice, according to Example 1 (*p< 0.05, **p< 0.01, ***p< 0.001).
**Figure 4** shows the (normalized) expression level of SOD1 (A), CAT (B), GPX3 (C), SOD3 (D), GST (E), GPX4 (F), HO-1 (G), GPX1 (H), GPX7(I) genes in vehicle-treated ("Vehicle"), and rhNGF-treated ("rhNGF") glaucoma mice, according to Example 1 (*p< 0.05, **p< 0.01, ***p< 0.001).
**Figure 5** shows the results of the signature of the Nrf2 pathway (A), and the Western blot analysis (B) of Nrf2 expression (GAPDH was the loading control), of vehicle-treated ("Vehicle"), and rhNGF-treated ("rhNGF") glaucoma mice, according to Example 1(*p< 0.05, **p< 0.01, ***p< 0.001).
**Figure 6** shows the signature of the M1 signature (A) and M2 signature (B) in vehicle-treated ("Vehicle"), and rhNGF-treated ("rhNGF") glaucoma mice, according to Example 1 (*p< 0.05, **p< 0.01, ***p< 0.001).
**Figure 7** shows the (normalized) expression level of iNOS (A), COX2 (also known as ptgs2) (B), IL12 (C), MHC II (D), CD86 (E), CD11b (also known as ITGAM) (F), IL18 (G), CCL2 (H), IL18r (I), CD14 (J), CXCL1 (K), mmp12 (L) genes, in vehicle-treated ("Vehicle"), and rhNGF-treated ("rhNGF") glaucoma mice, according to Example 1(*p< 0.05, **p< 0.01, ***p< 0.001).
**Figure 8** shows the (normalized) expression level of TGF-beta (A), ARG-1 (B), IGF1 (C), NGF (D), IL13 (E), GDNF (F), CD163 (G), CD206 (MRC1) (H), IL10rb (I), CSF1 (J), FGF1 (K), FGFR1 (L) genes, in vehicle-treated ("Vehicle"), and rhNGF-treated ("rhNGF") glaucoma mice, according to Example 1 (*p< 0.05, **p< 0.01, ***p< 0.001).
**Figure 9** shows the Western blot analysis of IL-1b, TNFα, IL-6 and IL-4 (GAPDH was the loading control) expression in vehicle-treated ("Vehicle"), and rhNGF-treated ("rhNGF") glaucoma mice, according to Example 1.

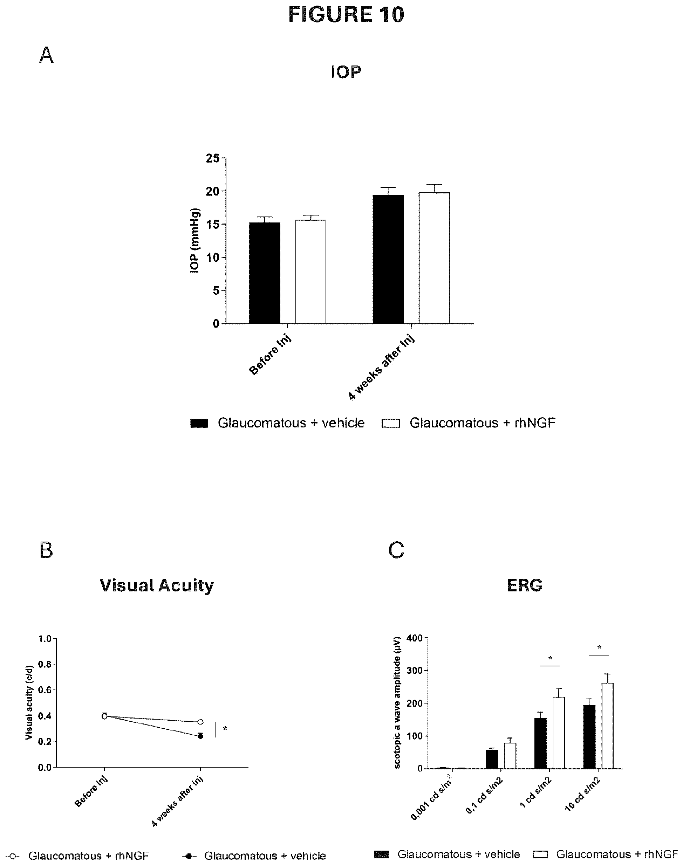

**Figure 10** shows the results of (A) intraocular pressure ("IOP", mmHg) and (B) visual acuity (c/d) analysis, before rhNGF or vehicle intravitreal injection ("Before inj."), and 4 weeks after rhNGF or vehicle intravitreal injection ("4 weeks after inj.") in 4-month-old animal groups, and the results of (C) scotopic a wave amplitude (µV) of ERG (at 0.001 cd·s/m², 0.1 cd·s/m², 1 cd·s/m² and 10 cd·s/m²) analysis of vehicle-treated glaucoma ("Glaucomatous + vehicle") and rhNGF-treated glaucoma ("Glaucomatous + rhNGF") 4-month-old animal groups, according to Example 1. Two-tailed Student's t-test. *p<0.05 vs. vehicle-treated glaucoma ("Glaucomatous + vehicle").

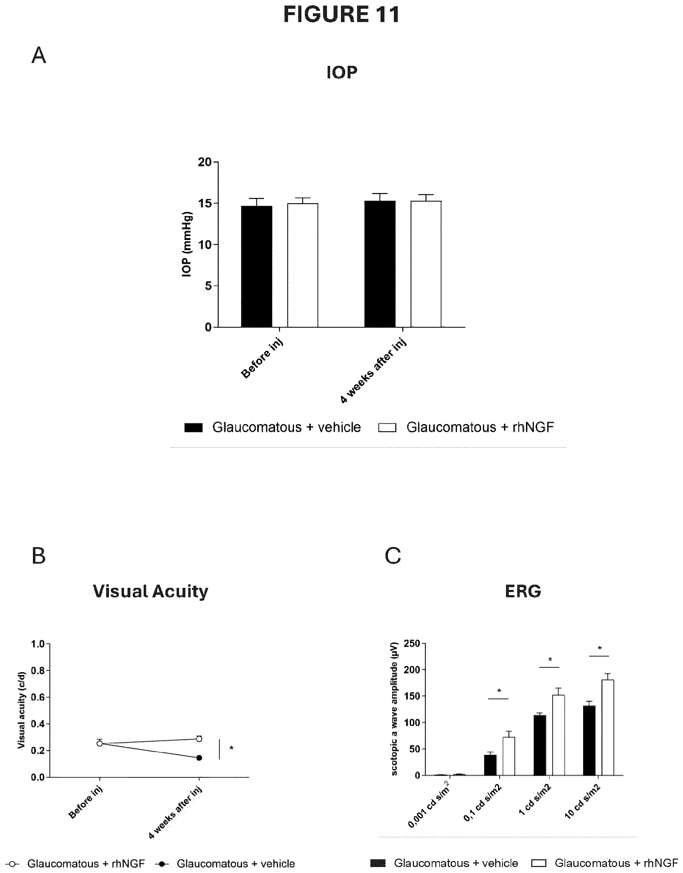

**Figure 11** shows the results of (A) intraocular pressure ("IOP", mmHg) and (B) visual acuity (c/d) analysis, before rhNGF or vehicle intravitreal injection ("Before inj."), and 4 weeks after rhNGF or vehicle intravitreal injection ("4 weeks after inj.") in 7-month-old animal groups, and the results of (C) scotopic a wave amplitude (µV) of ERG (at 0.001 cd·s/m², 0.1 cd.s/m², 1 cd·s/m² and 10 cd·s/m²) analysis of vehicle-treated glaucoma ("Glaucomatous + vehicle") and rhNGF-treated glaucoma ("Glaucomatous + rhNGF") 7-month-old animal groups, according to Example 1. Two-tailed Student's t-test. *p<0.05 vs. vehicle-treated glaucoma ("Glaucomatous + vehicle").

### DETAILED DESCRIPTION OF THE INVENTION

A first object of the invention is nerve growth factor (NGF) for use in slowing the progression of vision function impairment caused by glaucoma, and/or delaying the onset of vision function impairment or vision function loss caused by glaucoma in a subject, wherein said NGF is administered intravitreally to said subject.

Preferably, said subject is a human subject.

Preferably, the above slowing the progression of vision function impairment caused by glaucoma and/or delaying the onset of vision function impairment or vision function loss in a subject is in one or both eyes, more preferably in both eyes.

The term "slowing of the progression of vision function impairment" according to the present invention refers to achieving a slower progression of vision function impairment caused by glaucoma in the subject administered with NGF compared to same subject with no administration of NGF. Alternatively, "slowing of the progression of vision function impairment" according to the present invention refers to achieving a slower progression of vision impairment caused by glaucoma in the subject administered with NGF compared to a corresponding subject not administered with NGF, suffering from the same type and stage of glaucoma at the time of administration of NGF.

The term "delaying the onset of vision function impairment" according to the present invention refers to achieving a delay in the onset of a vision function impairment caused by glaucoma in the subject administered with NGF compared to same subject with no administration of NGF. Alternatively, "delaying the onset of vision function impairment" according to the present invention refers to achieving a delay in the onset of vision function impairment caused by glaucoma in the subject administered with NGF compared to a corresponding subject not administered with NGF, suffering from the same type and stage of glaucoma at the time of administration of NGF.

The term "delaying the onset of vision function loss" according to the present invention refers to achieving a delay in the onset of vision function loss caused by glaucoma in the subject administered with NGF compared to same subject with no administration of NGF. Alternatively, "delaying the onset of vision function loss" according to the present invention refers to achieving a delay in the onset of vision function loss caused by glaucoma in the subject administered with NGF compared to a corresponding subject not administered with NGF, suffering from the same type and stage of glaucoma at the time of administration of NGF.

According to a preferred aspect of the present invention,said visual function impairment or loss may be established by evaluating visual acuity, visual field index (VFI), mean deviation (MD) or pattern standard deviation (PSD), ganglion cell layer and retinal nerve fiber layer (RNFL) thickness in the subject.

Preferably, said visual function impairment or loss is determined by functional assessments, for example by best corrected visual acuity (BCVA) (measured in Early Treatment Diabetic Retinopathy Study [ETDRS] charts), Humphrey Visual Field (HVF) analysis, preferably using the SAP 24-2 SITA standard protocol; visual field index (VFI), mean deviation (MD) and pattern standard deviation (PSD), preferably assessed by Humphrey Visual Field Analyzer (HVFA) using the SAP 24-2 -SITA standard).

Preferably, said glaucoma is open-angle glaucoma, angle-closure glaucoma or congenital glaucoma. More preferably, said glaucoma is open-angle glaucoma.

Preferably, said glaucoma is in the early, intermediate or advanced stage, more preferably in the intermediate or advanced stage.

According to the present invention:
By "glaucoma in an early stage", it is meant a glaucoma wherein the mean deviation in the Humphrey 24-2 visual field (VF) test is better than -6dB is measured.

By "glaucoma in intermediate stage", it is meant a glaucoma wherein the mean deviation in the Humphrey 24-2 visual field (VF) test is between -6.01 and -12 dB.

By "glaucoma in advanced stage", it is meant a glaucoma wherein the mean deviation in the Humphrey 24-2 visual field (VF) test is between -12.01 and -20 dB.

By "glaucoma in late stage", it is meant a glaucoma wherein the mean deviation in the Humphrey 24-2 visual field (VF) test worse than -20.01 dB.

In a preferred aspect of the present invention, said subject suffers from glaucoma, preferably in the early, intermediate or advanced stage, and said NGF is for use in slowing the progression of vision function impairment caused by glaucoma in the subject, said NGF being administered intravitreally to the subject.

In another preferred aspect, said subject suffers from glaucoma, preferably in the early stage, and said NGF is for use in delaying the onset of vision function impairment caused by glaucoma in the subject, said NGF being administered intravitreally to the subject.

In another preferred aspect, said subject suffers from glaucoma, preferably in the early, intermediate or advanced stage, and said NGF is for use in delaying the onset of vision function loss caused by glaucoma in the subject, said NGF being administered intravitreally to the subject.

Preferably, said NGF is human NGF.

Preferably, said human NGF has the amino acid sequence of SEQ. ID NO.1 below.

SEQ. ID NO.1:
SSSHPIFHRGEFSVCDSVSVWVGDKTTATDIKGKEVMVLGEVNINNSVFKQYFFETKCRD
PNPVDSGCRGIDSKHWNSYCTTTHTFVKALTMDGKQAAWRFIRIDTACVCVLSRKAVR

Alternatively, said human NGF has the amino acid sequence of SEQ ID NO. 2 below:

SEQ ID NO. 2:
SSSHPIFHRGEFSVCDSVSVWVGDKTTATDIKGKEVMVLGEVNINNSVFKQYFFETKCRD
PNPVDSGCRGIDSKHWNSYCTTTHTFVKALTMDGKQAAWRFIRIDTACVCVLSRKAVRR
A

Alternatively, said human NGF is a mixture of NGFs having sequences of SEQ ID NO. 1 and SEQ ID NO. 2.

The human NGF of SEQ ID NO. 2 has an amino acid sequence that only differs from the NGF of SEQ ID NO. 1 for the presence of two additional amino acids at the C-terminus. Both forms of NGF are found in human cells and therefore are considered as wild type human NGF.

Therefore, when referring to "human NGF" or "wild-type human NGF" in the present application, it is meant a human NGF of SEQ ID NO. 1 or SEQ ID NO. 2 or mixtures thereof.

Preferably, said NGF is produced by recombinant DNA technology, preferably it is a human recombinant NGF (rhNGF). Methods of producing rhNGF are known to the person skilled in the art, for example those described in WO0022119A1 and WO2013092776A1.

Preferably, said NGF has a purity higher than 70%, more preferably higher than 80%, higher than 90%, higher than 95%, higher than 98% or higher than 99%. The purity of NGF may be determined by conventional means known to those skilled in the art, for example by HPLC analysis.

Preferably, said NGF is administered to said subject by intravitreal injection in an amount/eye per each administration between 0.1 µg and 20 µg, preferably between 0.5 µg and 10 µg, more preferably between 1 µg and 5 µg, even more preferably of 1, 1.5, 2 or 3 µg.

Preferably, the NGF for use according to the invention is administered to said subject by intravitreal injection in a volume per each administration comprised between 15 µl and 35 µl, more preferably between 20 µl and 30 µl, most preferably is 25 µl.

The administration schedule may vary depending on the patient and it is selected by the skilled person based on the characteristics of the subject to be treated, the severity of the disease and the tests carried out during the treatment to monitor response and progression of disease.

Preferably, the NGF for use according to the invention is administered to said subject more than once according to clinical evaluation of the patient. Preferably, the frequency of administration is not more often than once every four months, more preferably not more often than once every six months, even more preferably not more often than once a year.

A further object of the present invention is a pharmaceutical composition comprising said NGF, as disclosed above, and at least one pharmaceutically acceptable excipient, for use in slowing the progression of vision function impairment caused by glaucoma, and/or delaying the onset of vision function impairment or vision function loss caused by glaucoma in a subject, as described above, said pharmaceutical composition being administered intravitreally to said subject.

By "pharmaceutically acceptable excipient" as used herein, it is meant an excipient which is suitable for administration to the eye of a subject.

Preferably, said pharmaceutically acceptable excipient is selected from solvents, thickening agents, mucoadhesive agents, buffers, antioxidants and preservatives.

Said at least one pharmaceutically acceptable excipient may be selected from the group comprising: calcium chloride; carboxymethylcellulose sodium; DL-lactide and glycolide (50:50) copolymer 12000 acid; hyaluronate sodium; hydrochloric acid; magnesium chloride; magnesium stearate, microcrystalline cellulose; polyvinyl alcohol; potassium chloride; sodium acetate; sodium bicarbonate; sodium chloride; sodium hydroxide; sodium phosphate, dibasic, heptahydrate; sodium phosphate, monobasic, monohydrate; trisodium citrate dihydrate; citric acid monohydrate; sodium metabisulfite; sodium phosphate, dibasic, anhydrous; sodium phosphate, monobasic, anhydrous; sucrose; histidine; acetic acid; L-methionine; and trehalose.

Preferably, said pharmaceutical composition is a pharmaceutical liquid composition.

Said pharmaceutical liquid composition may be in form of a solution or suspension, preferably it is a solution.

Preferably, said pharmaceutical liquid composition contains a physiological saline solution (0.9% sodium chloride (NaCl)), as a major vehicle.

More preferably, said pharmaceutical liquid composition comprises, even more preferably consists of, NGF, preferably rhNGF, and physiological saline solution (0.9% sodium chloride (NaCl)).

Preferably, the pH of said pharmaceutical liquid composition is between 4.5 and 8.0, preferably around 7.

Preferably, the concentration of NGF in said pharmaceutical liquid composition is between 20 µg/ml and 180 µg/ml, more preferably between 40 µg/µl and 140 µg/µl, even more preferably selected from 60 µg/ml and 120 µg/ml.

Preferably, the volume of said pharmaceutical liquid composition is comprised between 15 µl and 35 µl, more preferably between 20 µl and 30 µl, most preferably is 25 µl.

The pharmaceutical liquid composition according to the invention may be suitably formulated using appropriate methods known in the art or by the method disclosed in Remington's Pharmaceutical Science (recent edition), Mack Publishing Company, Easton Pa.

In a further aspect, the present invention relates to a method for slowing the progression of vision function impairment caused by glaucoma, and/or delaying the onset of vision function impairment or vision function loss caused by glaucoma in a subject, as above described, comprising administering to the subject NGF, as described above, in a therapeutically effective amount by intravitreal injection.

The invention will be further described in the following examples, which do not limit the scope of the invention defined in the claims.

### EXPERIMENTAL SECTION

### Example 1

The pharmaceutical efficacy in treating glaucoma of intravitreal injections of rhNGF (SEQ ID No:1) on eyes of DBA/2J (Strain #:000671, obtained from the European distributor of Jackson Laboratories Mice (Charles Rivers Laboratories, Wilmington, MA, USA) mice was tested, according to the below.

Mice were housed in macrolon cages with filter hoods, in a room where the air is continuously filtered, thereby avoiding contamination. During experiments, paired animals were caged at a constant temperature with a day/night cycle of 12/12 hours.

Animals received water (control tap water) and nutrition ad libitum. Animal protocol is approved by the Italian ministry of health (approval code CE5C5.34) and performed by Invivex SAS with animal protocol approved by the Animal Studies Committee of Languedoc Roussillon (Reference Number: D3417223, APAFIS#23920-2020020320279696v3). Animal health will be examined every day to ensure that only animals in good health enter the testing procedures and follow up the study.

### Animal models and treatments:

DBA/2J mice are widely used as an *in vivo* model for the study of glaucoma, as they spontaneously develop glaucoma in a progressive and age-dependent manner (Amato et al, Cells 2023 (12), 1272).

Accordingly, to analyze the therapeutic effect of rhNGF at various stages of development of the pathology, two groups of animals were treated: a mice group of 4 months of age, and a mice group of 7 months of age.

In particular, the glaucoma condition of said 4-5 months mice group corresponds to the "early stage" glaucoma of human subjects as defined in the present invention; the glaucoma condition of said 6-9 months mice group corresponds to the "intermediate stage" and "advanced stage" glaucoma of human subjects as defined in the present invention.

Said groups were treated by intravitreal administration of a rhNGF solution (consisting of 1 µg/µl rhNGF, 0.9% saline (sodium chloride) solution), or a vehicle solution (consisting of 0.9% saline (sodium chloride) solution), according to the following:

### - Early stage of glaucoma:

- a group of 10, 4-month-old DBA/2J mice received a single intravitreal injection of the rhNGF solution ("Glaucomatous + rhNGF");
- a group of 10, 4-month-old DBA/2J mice received a single intravitreal injection of the vehicle solution ("Glaucomatous + vehicle").
- *Intermediate and advanced stages of glaucoma:*
- a group of 10, 7-month-old DBA/2J mice received a single intravitreal injection of the rhNGF solution ("Glaucomatous + rhNGF");
- a group of 10, 7-month-old DBA/2J mice received a single intravitreal injection of a vehicle solution ("Glaucomatous + vehicle").

All said DBA/2J mice were diagnosed with glaucoma, and specifically selected for an IOP ≥ 11 mmHg.

In order to perform intravitreal administrations, anesthesia was performed with an intraperitoneal injection of ketamine [50 mg/kg] /xylazine [10 mg/kg] mixture; then superior nasal region of the eye of the animal was exposed and a glass needle was inserted at a 45° angle through the sclera into the vitreous body. A small amount of vitreous was rid through the puncture hole from the posterior chamber by the eyeball mild pressure. Then, 1 µl of the above rhNGF solution or vehicle solution was injected into the posterior chamber and the needle was kept in place for few seconds.

In particular, a single injection of rhNGF/Vehicle (1 µg/eye, 1 µl per eye) solution has been injected only into the left eye of each mice.

After intravitreal injection, a drop of ophthalmic Tobrex solution was applied on the mouse eye to prevent infection.

### Analysis

To assess if intravitreally administered rhNGF could exert any effects of retinal cells, the following analysis was performed on the eyes of said animal model.

### Eye sampling

At the end of the intravitreal administration, all mice were euthanized. Following, half of the animals (n=5 animals/group/analysis) were used for histological and IHC analysis and the other half for RNA analysis and Western Blot.

The connective tissue in the orbital cavity surrounding the eye was cut. The optic nerve and any remaining tissue were also cut to extract the intact eyeball. Five eyes per group were quickly frozen in liquid nitrogen, and stored at -80 °C. The five remaining eyes per group were placed in a petri dish containing fresh PBS. A small incision was performed just posterior to the border between the iris and the sclera allowing the PFA penetration. Then, each eye was fixed individually with 4 % PFA in 0.1 M phosphate buffer (pH 7.3) at 4 °C overnight. After eye fixation, samples were incubated in sucrose 30 % in PBS during 48 hours at 4 °C and finally, each eye was individually embedded in OCT cryoconservation medium for Immunohistochemistry (IHC) analysis.

### Immunohistochemistry

Immunohistochemistry (IHC) was performed on OCT tissue blocks. The primary antibodies used were as follow: IBA1 (# 019-19741) were purchased from FUJIFILM Wako Chemicals U.S.A. Corporation, GFAP (#3670 were purchased from cell signalling technologies). Immunoreactions were visualised using Polyview Plus HRP (anti-rabbit) reagent (Enzo life science #ENZ-ACC103-0150) and sections were counterstained using Mayer's haematoxylin (Emallume Carazzi Bio-Optica, Milano, ITA). TUNEL assay (Terminal deoxynucleotidyl transferase dUTP nick end labeling) was performed by using TUNEL in situ apoptosis kit (HRP-DAB method) according to manufacturer instruction (Immunuological Sciences, Cat Number: IKA331). Histological images were acquired using a Nikon Eclipse E200 microscope equipped with a Leica DFC310 FX Digital Camera. The percentage of positive area was quantified using the QuPath Software.

### Western Blot

Western Blots were performed using ECL (Amersham) as previously described (REF: PMID: 35805202). Proteins were extracted using RIPA buffer (1x phosphate buffered saline, 1% NP40, 0.5% sodium deoxycholate, 1% SDS, 0.1 mM PMSF, 1 µg/ml aprotinine, 0.1 M Na3VO4) supplemented with complete mini EDTA-free protease inhibitors (Roche Molecular Biochemicals, Basel Switzerland). The antibodies used were as follow: TNF alfa (#11948); IL-1-BETA (#12242); IL6 (#12912); GAPDH (#5174); NRF2 (#20733); were purchased from Cell Signalling Technology, while IL4 (#PA5115416) were purchased from Thermo Fisher Scientific. Membranes were then incubated with specific horseradish peroxidase-conjugated secondary antibodies (Santa Cruz Biotech). Protein bands were visualised using a chemiluminescent detection system (Thermo Scientific, Rockford, USA).

### RNA Extraction and quantitative RNAseq

Total RNA was isolated from total retina of mice using the mirVana isolation kit (Invitrogen, Thermo Fisher Scientific, Waltham, MA, USA), according to the manufacturer's protocol. The concentration of RNA was assessed by Qubit Fluorometer (Invitrogen, Thermo Fisher), and RNA samples were stored at -80 °C until use.

For RNAseq analysis, 250 ng of each RNA sample were sent to Genomix4life S.R.L. of Baronissi (Italy), which performed both quality control test and NGS experiments.

### Bioinformatic analysis

Data analysis of miRNA cards was performed using QuantStudio Software v 1.3 and Expression Suite software v 1.3 (Thermo Fisher). MiRNA expression levels (RQ, relative quantification) were assessed by comparative assay (2-ΔΔCt) and data were normalized using global normalization method. A manual check focused on PCR amplification plots profiles was also performed and only miRNAs with good amplification curves were retained.

For RNAseq experiments, prior to further analysis, the fastq files from Illumina were assessed for quality by using FastQC tool (v0.12.0) and trimmed for adapter sequences and low base call quality (Phred score < 30 at ends) with cutadapt (v4.4). Only the reads with length more than 10 were retained. The trimmed reads were mapped on reference genome using STAR (v2.7.10b). GRCm39 was used as the reference genome and the comprehensive gene annotations on the primary assembly from Gencode (Release M34) used as gene annotation. The quantification of transcripts expressed for each sequenced sample was performed using featureCount (version 2.0.3). Both STAR and featureCount executions were performed using the standard parameters. Differential gene expression analysis was performed using the DESeq2 package (v1.44.0) and differentially expressed protein coding genes were considered significant if their p value after Benjamini & Hochberg correction was < 0.001. The enrichment of GO terms on the up, down and all the differentially expressed genes was evaluated using clusterProfiler (v4.12.3). For the enrichment analysis, the background set consisted of all coding genes detected in either Vehicle or rhNGF samples. Conversion from mouse gene symbols to their human one-to-one orthologs was performed by using the nichenetr package (v 2.1.5). GO terms were considered significantly enriched if their p value after Bonferroni correction was <0.05. Both the differential and enrichment analyses, along with the corresponding plots, were performed using R (v4.0). The NRF2, M1 and M2 and RGC gene signatures (as disclosed at: https://www.gsea-msigdb.ora/gsea/msigdb/index.jsp) were scaled according to the Z-scores, and the average values were used as the scores for the respective gene signatures.

### Intraocular pressure (IOP) measure

Tonolab system (Icare LAB tonometer, TonoLab, Vantaa, Finland) was used to quantitatively assess the intraocular pressure in rodents at indicated time points. The animals were immobilized with the left hand. Then, the tonometer was brought near the mouse eye with the groove in a horizontal position. The distance between the tip of the probe and the mouse cornea was kept at 1 to 3 millimeters. Then, the tip of the probe was advanced until contact with the central cornea, and the measuring button was pressed six times. Once the six correct single measurements were done, the IOP was shown on display. Final IOP reading was an average of 4 mean single readings. IOP measurements were performed before intraocular injection (baseline) and at endpoint.

### Visual acuity measure

The visual acuity of the above tested animals was determined by the following optometry test. The animals were individually placed on a platform in the center of a cylinder. Right eye was closed using histoacryl. Animals were adapted to the environment for 5 min. The mouse number and condition were selected on the software. The step size of the stimulus was selected manually. The stimulation conditions were: 0.05 c/d sinusoidal for the spatial frequency, speed 12 d/s and temporal frequency 0.6 Hz. The reflexive head and neck movements were noted for each animal. The improvement of the spatial frequency measured in cycles/degree (c/d) was measured. Each animal performed 3 trials at 5 minutes intervals. For each day, values from the 3 trials were averaged for each animal and then averaged for each treated group.

### Electroretinoqraphy (ERG)

Mice were anesthetized using ketamine [50 mg/kg] /xylazine [10mg/kg] mixture individually. Eye was treated with 1% atropine sulphate, 2.5% phenylephrine hydrochloride and 0.5% proparacaine hydrochloride, allowing drops to sit on the eyes for ~2 min before wicking with a cotton swab and applying the next drop. Then, the mouse was positioned on the heated platform. Right eye was closed using histoacryl. The ground needle electrode was placed in the base of the tail, reference needle electrode subdermally between the eyes, a drop of 2.5% hypromellose was applied, and the contact lens electrodes was placed onto the cornea. The scotopic a-wave (dark-adapted) ERG measures were performed at 0.001 cd s/m2; 0.1cd s/m2; 1 cd s/m2; and 10 cd s/m2 with pulse frequency: 0.2 Hz, sample frequency: 1.000 Hz, trial pre-trigger time: 20 msec and trial post-trigger time: 250 msec (Ganzfeld ColorDome, Diagnosys, catalogue number: D125). The amplitude values of left eyes were averaged automatically for each animal and then averaged for each group. At the end of the measure, electrodes were removed from the mouse and the animal was placed in a clean cage on top of a heat pad until recovering. The day before ERG analysis, all mice were placed in a light-proof dark room allowing them to dark-adapt overnight.

### Statistical analysis

All data are presented as mean ± standard deviation (SD) unless stated otherwise. Statistical significance was determined by two-tailed Student's t-test. p-values < 0.05 were considered statistically significant. Analyses used GraphPad Prism version 9.0, GraphPad Software (San Diego, CA).

### - Results

### - Effects of the rhNGF treatment on histological parameters of glaucomatous eyes (glaucoma at intermediate and advanced stages).

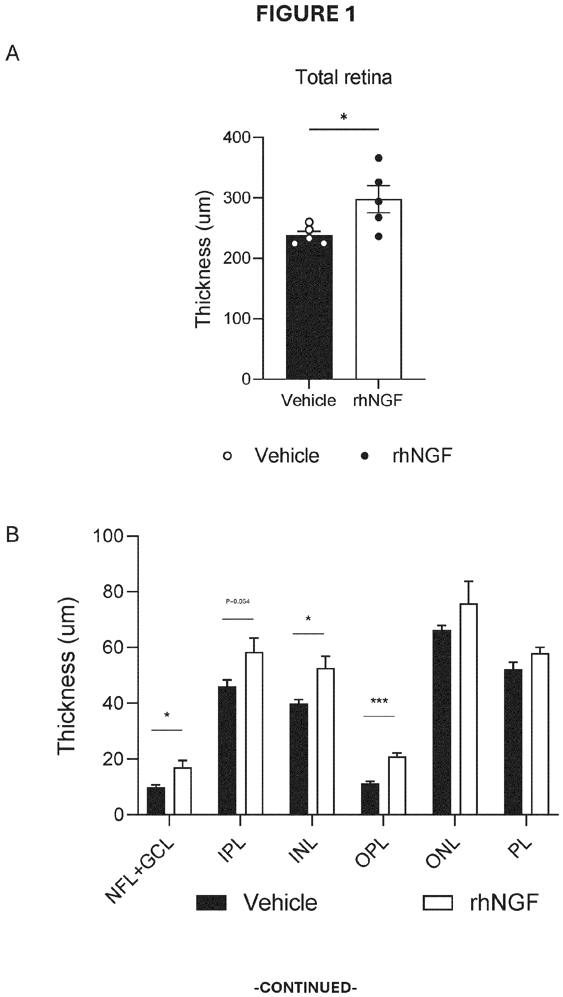

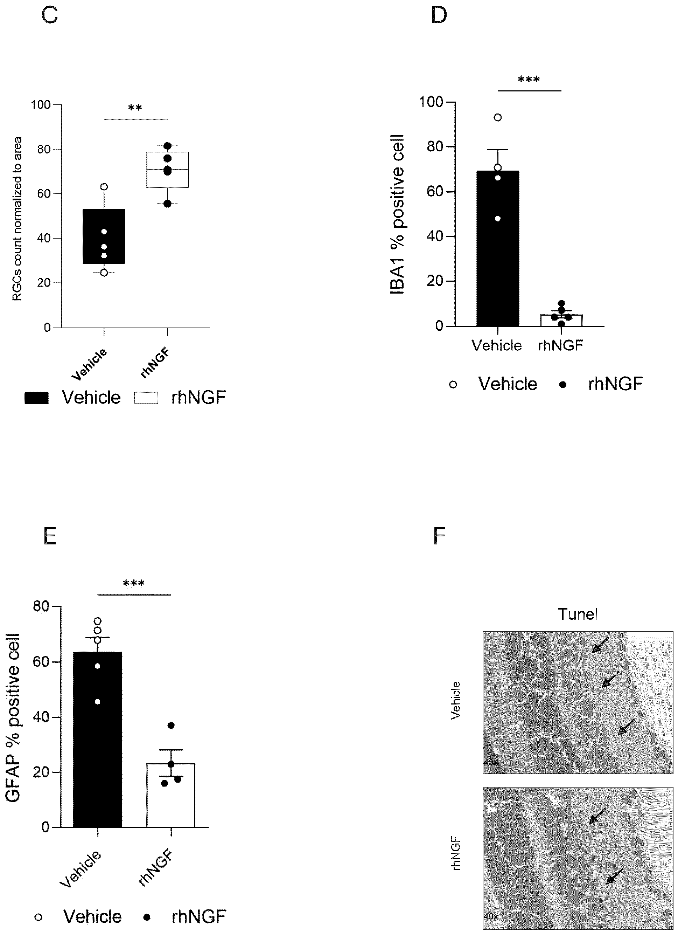

The results of the histological analysis of the 7-month-old DBA/2J mice group are reported in Figures 1A, 1B and 1C.

In particular, Figure 1A shows that the rhNGF treatment causes an increase of the thickness of the total retina, and Figure 1B shows that such an increase is also reported for each of the retinal layer, namely nerve fiber layer (NFL) and ganglion cell layer (GCL), inner plexiform layer (IPL), inner nuclear layer (INL), outer plexiform layer (OPL), outer nuclear layer (ONL), photoreceptor layer (PL), in respect of the vehicle-treated mice.

Figure 1C shows that the rhNGF treatment also increases the RGCs count, in respect of the vehicle-treated mice.

Moreover, by IHC analysis, a lower activation of inflammatory markers Iba-1 and GFAP following rhNGF treatment in said glaucoma animal model was observed, as shown in Figure 1D and Figure 1E, respectively.

Finally, to assess if rhNGF also affects the apoptosis, a TUNEL test (Terminal deoxynucleotidyl transferase dUTP nick end labeling), a known apoptosis-positive staining, in the inner nuclear layer (INL) was carried out, and the results are reported in Figure 1F.

The rhNGF treatment also causes a reduction in TUNEL positivity, in respect of the vehicle-treated mice.

These results demonstrate a cytoprotective effect of rhNGF, and a significant reduction in reactive gliosis of the rhNGF-treated glaucomatous mice eyes.

### - Effects of the rhNGF treatment on the RGC viability of glaucomatous eyes (glaucoma at intermediate and advanced stages).

In order to further assess the cytoprotective role played by rhNGF in respect of the 7-month-old DBA/2J mice group eye cells, the expressions of the most known (specific) marker of RGC vitality were analyzed by RNA sequence analysis according to the above.

The selected markers were the following: POU Class 4 Homeobox 1 (Pou4f1/Brn3a), Synuclein Gamma (Sncg), RNA-binding protein with multiple splicing (Rbpms), Ubiquitin carboxyl-terminal hydrolase isozyme L1 (UCHL1), Tubulin Beta 3 Class III (Tubb3), RNA-binding protein with multiple splicing 2 (Rbpms2), ELAV-like protein 3 (Elavl3) and Neurofilament Light Chain (Nefl).

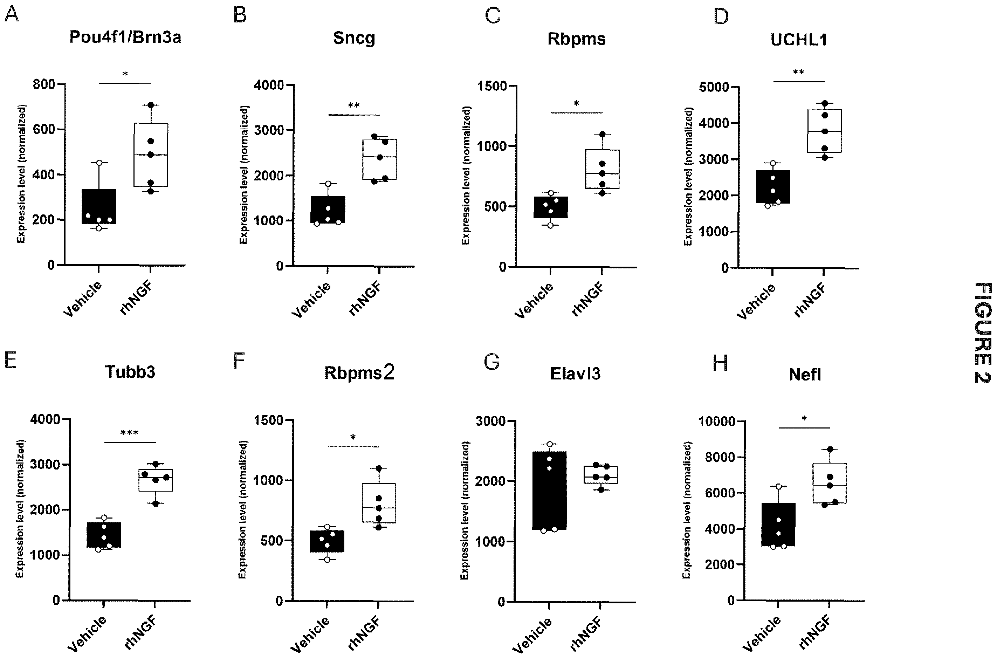

The results of the expression analysis of each of the above markers are reported in Figure 2A-2H, respectively.

The analysis highlighted a statistically significant increase in Pou4f1, Sncg, Rbpms, UCHL1, Tubb3, Rbpms2 and Nefl, thus showing that the rhNGF treatment increases the expression of the viability markers of RGCs in glaucomatous eyes.

### - Effects of the rhNGF treatment on oxidative stress of glaucomatous eyes (glaucoma at intermediate and advanced stages).

In order to assess if the rhNGF treatment would also have an effect of oxidative stress level in the 7-month-old DBA/2J mice group , the expression of the pro-oxidative markers nitric oxide synthase 1 (NOS1), nitric oxide synthase 2 (NOS2) and nitric oxide synthase 3 (NOS3), was analyzed by RNA sequence analysis according to the above.

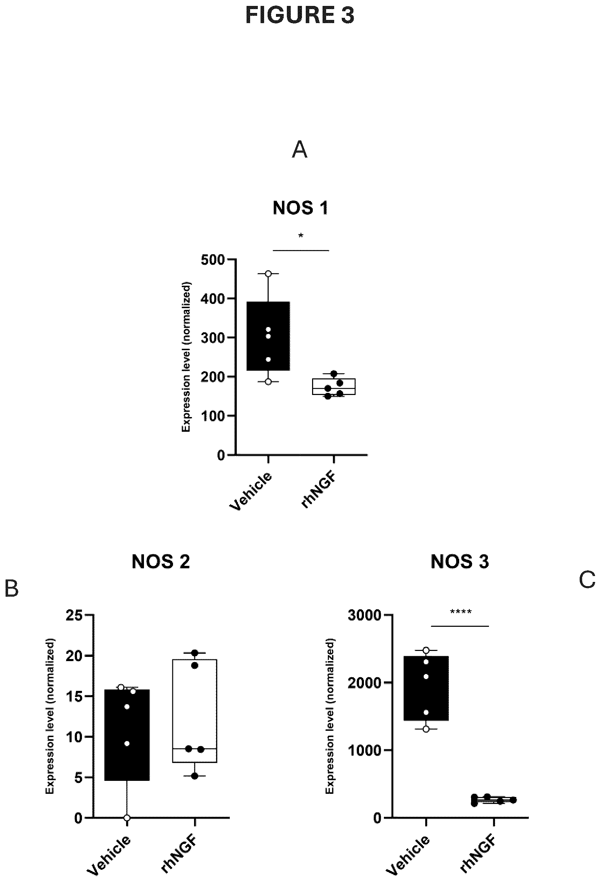

Figure 3A shows the results of the NOS1 expression, Figure 3B shows the results of the NOS2 expression, Figure 3C shows the results of the NOS3 expression.

As shown in these Figures, the expression of NOS3 was drastically reduced in the rhNGF-treated animals, and the expression of NOS1 was particularly reduced in that animals, in respect to the vehicle-treated mice.

The expression of antioxidant markers was also analyzed by RNA sequence analysis according to the above.

In particular, the analyzed antioxidant markers were: Superoxide Dismutase 1 (SOD1), catalase (CAT), Glutathione Peroxidase 3 (GPX3), Superoxide Dismutase 3 (SOD3), Glutathione S-transferase (GST), Glutathione Peroxidase 4 (GPX4), Histone Deacetylase 1 (HO-1), Glutathione Peroxidase 1 (GPX1), Glutathione Peroxidase 7 (GPX7).

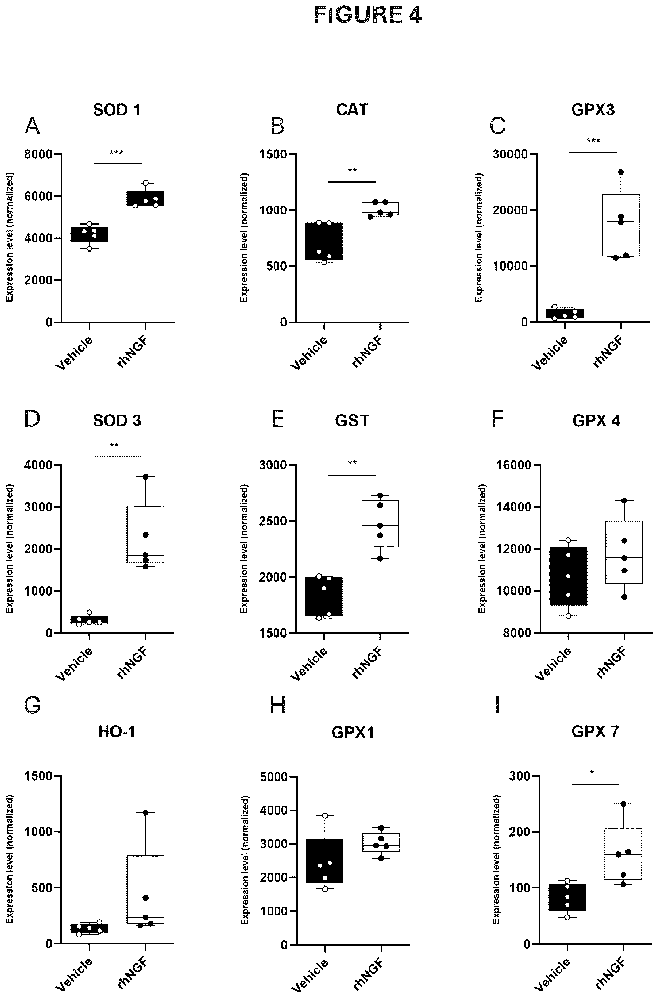

The results of the expression analysis of each of the above markers are reported in Figure 4A-4I, respectively.

Along with the down-regulation of the pro-oxidative markers as shown above, the rhNGF treatment causes an up-regulation of said antioxidant markers in the retina of rhNGF-treated glaucoma mice, in respect to the vehicle-treated mice.

Then, an analysis of the signature of the Nrf2 pathway, a transcription factor that plays a key role in the modulation of antioxidant genes, was carried out by RNA sequence analysis and a western blot was carried out according to the above.

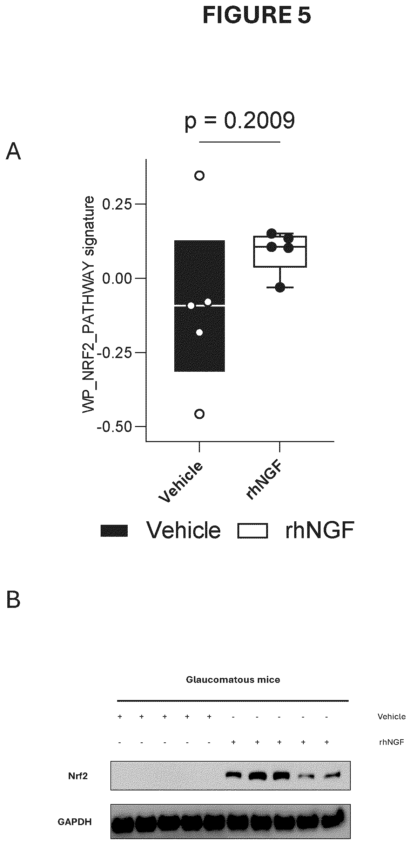

The results of the signature of the Nrf2 pathway are reported in Figure 5A, and the western blot results are reported in Figure 5B (GAPDH was the loading control).

As shown in these Figures, the signature of the Nrf2 pathway was increased in rhNGF-treated glaucoma mice, a data also confirmed by the western blot analysis of Nrf2 protein expression.

These results demonstrate that the rhNGF treatment exerts a significant neuroprotective effect on intermediate and advanced stage glaucoma RGCs, which is due to the modulation by NGF of the oxidative stress induced by the glaucoma disease.

### - Effects of the rhNGF treatment on the inflammation of glaucomatous eyes (glaucoma at intermediate and advanced stages).

Considering that immunohistochemical analysis showed a decrease in reactive gliosis markers Iba-1 and GFAP expression, and considering the well-known role of neuroinflammation in the induction of retinal damage in glaucoma at the intermediate and advanced stages, the expression of markers responsible for microglia polarization in the pro-inflammatory (M1) and (M2) phenotypes were analyzed by out by RNA seq analysis.

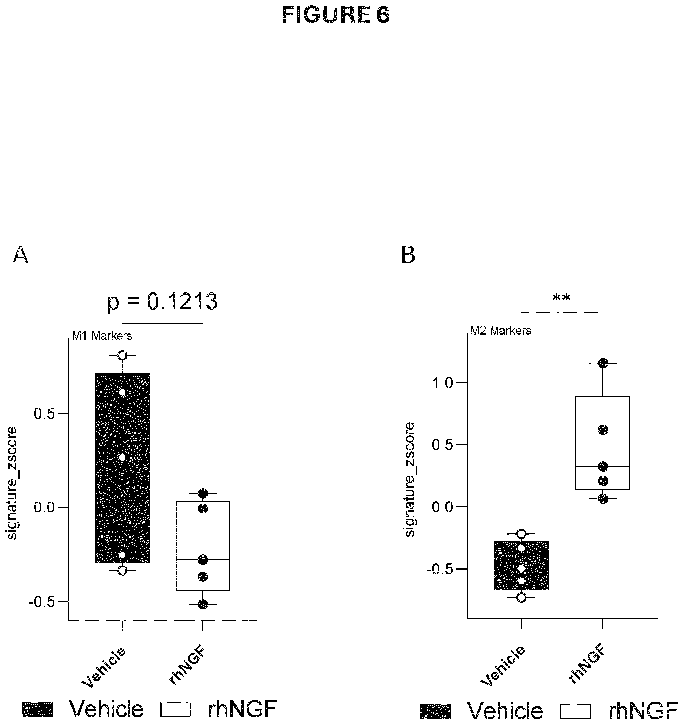

The results of the analysis of M1 pro-inflammatory signature are shown in Figure 6A, and the results of the analysis of M2 anti-inflammatory signature are shown in Figure 6B.

As shown in these Figures, the analysis of M1 and M2 signatures showed a trend of reduction of the M1 signature (Figure 6A) and a significant increase of the M2 signature in rhNGF-treated mice (Figure 6B), in respect to the vehicle-treated mice.

This data is also supported by the analysis of the expression of individual pro-inflammatory markers of M1 signature, and the expression of individual anti-inflammatory markers of M2 signature, as reported below.

Said pro-inflammatory markers were: Inducible nitric oxide synthase (iNOS), Cyclooxygenase-2 (COX2, also known as prostaglandin-endoperoxide synthase 2 , ptgs2), Interleukin 12 (IL12), Major histocompatibility complex class II (MHC II), Cluster of Differentiation 86 (CD86), Cluster of differentiation molecule 11b (CD11b (ITGAM)), Interleukin 18 (IL18), chemokine (C-C motif) ligand 2 (CCL2), Interleukin 18 Receptor (IL18r), cluster of differentiation 14 (CD14), Chemokine (C-X-C motif) ligand 1 (CXCL1), matrix metalloproteinase 12 (mmp12).

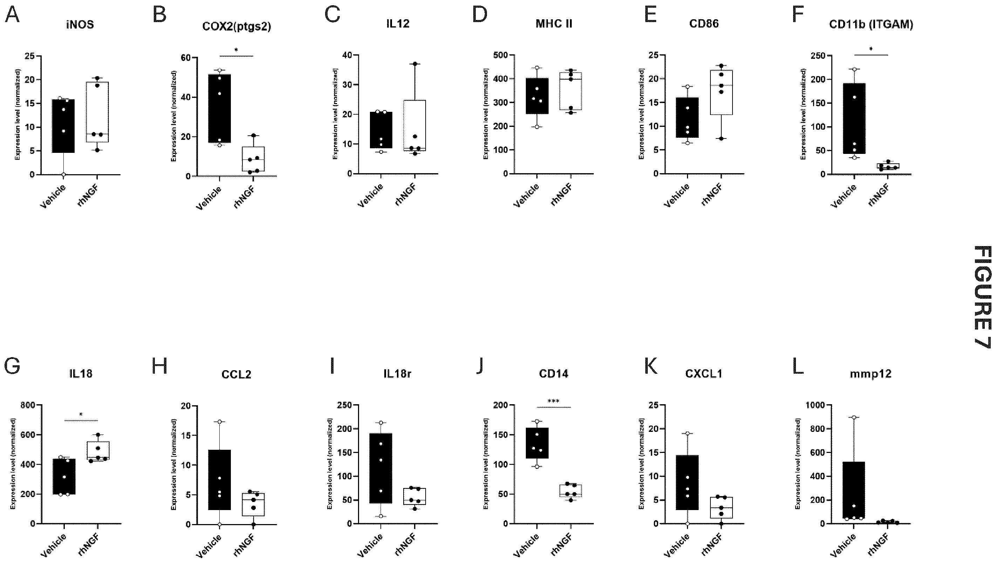

The results of the expression analysis of each of the above markers are reported in Figure 7A-7L, respectively, which show that the rhNGF treatment reduces the expression of most of the analyzed markers, in particular CD14, in respect to the vehicle-treated mice.

Said anti-inflammatory markers were: Transforming growth factor-beta (TGF-beta), Arginase-1 (ARG-1), Insulin-like growth factor 1 (IGF1), Nerve growth factor (NGF), Interleukin 13 (IL13), Glial cell line-derived neurotrophic factor (GDNF), Cluster of Differentiation 163 (CD163), Cluster of Differentiation 206 (CD206 (MRC1)), Interleukin 10 receptor subunit beta (IL10rb), Colony stimulating factor 1 (CSF1), Fibroblast growth factor 1 (FGF1), Fibroblast growth factor receptor 1 (FGFR1).

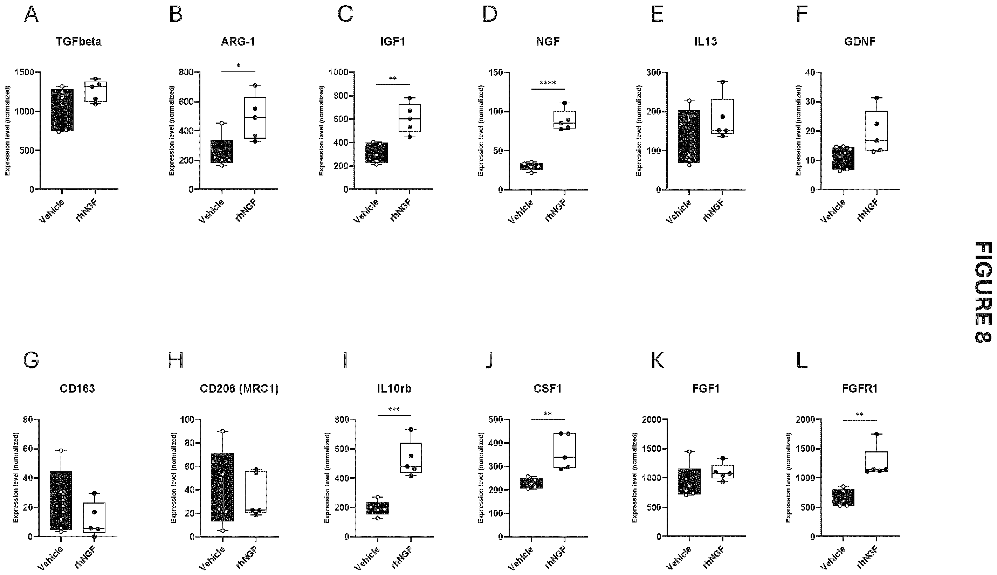

The results of the expression analysis of each of the above markers are reported in Figure 8A-8L, respectively, which show that the rhNGF treatment enhances the expression of most of the analyzed markers, in particular IL-10rb, NGF, and FGFR1, in respect to the vehicle-treated mice.

Similar results were obtained by analyzing the expression of pro-inflammatory markers Interleukin-1 beta (IL-1β), Interleukin 6 (IL-6) and Tumor necrosis factor-alpha (TNF-α), and of the anti-inflammatory marker Interleukin 4 (IL-4), by western blot analysis carried out as above disclosed (GAPDH was the loading control).

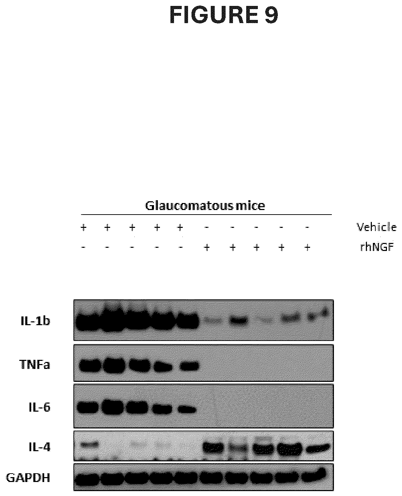

The results of the western blot are reported in Figure 9.

The rhNGF treatment clearly showed a significant reduction in the levels of pro-inflammatory IL-1β, IL-6 and TNF-α, suggesting an important effect of the molecule in reducing the pro-inflammatory response, and a significant increase in the level of IL-4, a cytokine with anti-inflammatory activity.

These data clearly show and confirm a significant cytoprotective anti-inflammatory role of rhNGF in intermediate and advanced stage glaucoma eyes.

### - Effect of the rhNGF treatment on IOP, visual acuity and retinal function in early, intermediate and advanced stages of glaucoma.

The above-reported group of 4-month-old mice allowed to evaluate the effect of said intravitreal injection of rhNGF on an early glaucoma stage mouse model of glaucomatous pathology.

Surprisingly, no effect of rhNGF injection on IOP was observed in the rhNGF treated group ("Glaucomatous + rhNGF"), compared to the IOP vehicle-treated group ("Glaucomatous + vehicle"), both before the intravitreal injection and 4 weeks after the intravitreal injection as shown in Figure 10A.

The results of the visual acuity test are reported in Figure 10B.

The rhNGF-treated group ("Glaucomatous + rhNGF") showed a significantly improved visual acuity than the vehicle-treated group ("Glaucomatous + vehicle"), therefore showing a visual improvement effect of rhNGF in the visual acuity.

The results of the ERG analysis are reported in Figure 10C.

The intravitreal administration of rhNGF demonstrated an unexpected enhancement in retinal function in rhNGF-treated group ("Glaucomatous + rhNGF"), compared to vehicle-treated group ("Glaucomatous + vehicle").

The above-reported group of 7-month-old mice allowed to clarify how intravitreal rhNGF influences the molecular and cellular events of an already partially damaged glaucomatous retina.

Surprisingly, even in this glaucoma animal model, no effect of rhNGF injection on IOP was observed in the rhNGF treated group ("Glaucomatous + rhNGF"), compared to the IOP vehicle-treated group ("Glaucomatous + vehicle"), both before the intravitreal injection and 4 weeks after the intravitreal injection as shown in Figure 11A.

The results of the visual acuity test are reported in Figure 11B.

As shown in above-reported results of the treatment of the early stage of glaucoma, the rhNGF-treated group ("Glaucomatous + rhNGF") showed significantly improved visual acuity than the vehicle-treated group ("Glaucomatous + vehicle"), in the intermediate and advanced stages of glaucoma, too.

The results of the ERG analysis are reported in Figure 11C.

The intravitreal administration of rhNGF demonstrated an unexpected and significant enhancement in retinal function in rhNGF-treated group ("Glaucomatous + rhNGF"), compared to vehicle-treated group ("Glaucomatous + vehicle"), even at 0.1 cd s/m².

## Claims

1. Nerve growth factor (NGF) for use in slowing the progression of vision function impairment caused by glaucoma, and/or delaying the onset of vision function impairment or vision function loss caused by glaucoma in a subject, wherein said NGF is administered intravitreally to said subject.

2. NGF for use as claimed in claim 1, wherein the amount/eye per each administration between 0.1 µg and 20 µg, preferably between 0.5 µg and 10 µg, more preferably between 1 µg and 5 µg, even more preferably of 1, 1.5, 2 or 3 µg.

3. NGF for use as claimed in claim 1 or 2, said NGF is human NGF, more preferably it is recombinant human NGF.

4. NGF for use as claimed in claims 1 to 3, wherein said human NGF consists of the amino acid sequence of SEQ ID No. 1 or SEQ ID No. 2 or mixtures thereof.

5. A pharmaceutical composition comprising nerve growth factor (NGF), and at least one pharmaceutically acceptable excipient, for use in slowing the progression of vision function impairment caused by glaucoma, and/or delaying the onset of vision function impairment or vision function loss caused by glaucoma in a subject, said pharmaceutical composition being administered intravitreally to said subject.

6. A pharmaceutical composition for use as claimed in claim 5, wherein said NGF is present in the composition at a concentration between 20 µg/ml and 180 µg/ml, preferably between 40 µg/µl and 140 µg/µl, more preferably selected from 60 µg/ml and 120 µg/ml.

7. A pharmaceutical composition for use as claimed in claim 5 or 6, wherein said NGF is human NGF, preferably it is recombinant human NGF.

8. A pharmaceutical composition for use as claimed in claims 5 to 7, wherein said human NGF consists of the amino acid sequence of SEQ ID No. 1 or SEQ ID No. 2 or mixtures thereof.

9. NGF for use as claimed in claims 1 to 4, or said pharmaceutical composition for use as claimed in claims 5 to 8, wherein said glaucoma is open-angle glaucoma, angle-closure glaucoma or congenital glaucoma, preferably open-angle glaucoma.

10. NGF for use as claimed in claims 1 to 4, or claim 9, or said pharmaceutical composition for use as claimed in claims 5 to 8, or claim 9, wherein said glaucoma is in the early, intermediate or advanced stage, preferably in the intermediate or advanced stage.
